# EUROPEAN PATENT SPECIFICATION

(11) **EP 0 466 909 B1**
(45) Date of publication and mention of the grant of the patent: **28.06.1995**
(21) Application number: 91904573.2
(22) Date of filing: 05.02.1991
(51) Int. Cl.: A61K 31/195

(54) **THYROID HORMONE FOR CARDIAC TREATMENT**
SCHILDDRÜSENHORMONE ZUR KARDIALEN BEHANDLUNG
HORMONES THYROIDIENNES POUR TRAITEMENT CARDIAQUE

(30) Priority: 05.02.1990 US 475360; 19.03.1990 US 495354
(43) Date of publication of application: 22.01.1992
(73) Proprietor: BRITISH TECHNOLOGY GROUP (USA) INCORPORATED, Gulph Mills, PA 19406 (US)
(72) Inventor: RUBIN, Leo, Suffern, NY 10901 (US)
(74) Representative: Percy, Richard Keith
(86) International application number: US9100750
(87) International publication number: WO9111181

(56) References cited:
- WO-A-89/07454
- FR-A- 2 354 101
- Eur. J. Cardio-thorac Surg. (1989) 3:140-145; Novitzky et al.
- Merck Manual 1987, page 501
- Patent Abstracts of Japan, vol. 12, no. 306 (C-522) (3153), 19 August 1988 & JP, A, 6379824
- Rote Liste, 1989, Editio Cantor, (Aulendorf/Württ., DE), no. 73001-73013 see no. 73008
- Acta Med. Scand., vol. 222, 1987, B. Hylander et al.: "Long-term ECG recordings in thyroxine-substituted hypothyroid subjects", pages 429-432 see the whole article
- Annales & Endocrinologie, vol. 44, no. 4, 1983, Masson, (Paris, FR) U. Paschen et al.: "Alternation in thyroid hormone concentration during and after coronary bypass operation", pages 239-242 see the whole article
- Diabetes, vol. 37, November 1988, H. Xiang et al.: "Effect of myo-inositol and T3 on myocardial lipids and cardiac function in streptozocin-induced diabetic rats", pages 1542-1548 see the whole article
- American Journal of Physiology, vol. 226, no. 1, January 1974, (US), M.J. Goodkind et al.: "Effect of thyroxine on ventricular myocardial contractility and ATPase activity in guinea pigs", pages 66-72 see the whole article

## Description

This invention relates to the use of thyroid hormones and related compounds to facilitate cardiac resuscitation and enhance cardiac function.

Cardiac arrest occurs when there is electrical and mechanical dysfunction in the heart. The survival of cardiac arrest depends on timely defibrillation and the administration of proper medications. Standard treatments for cardiac arrest include the administration of various drugs appropriate to the variety of situations in which cardiac arrest occurs. However, a more effective treatment is clearly required in view of the current low survival rate and high annual morbidity from cardiac arrest.

Thyroid hormones include thyroxine [O-(4-hydroxy-3,5-diiodophenyl)-3,5-diiodotyrosine, T4], which is hereinafter referred to as thyroxine, and 3,5,3' triiodothyronine [O-(4-hydroxy-3-iodophenyl)-3,5-diiodotyrosine, T3], which is hereinafter referred to as triiodothyronine. Triiodothyronine is qualitatively similar to thyroxine in its biological effect but is more potent on a molar basis. Although some triiodothyronine is synthesized in the thyroid gland, the majority of the naturally occurring compound is synthesized through the metabolic conversion of thyroxine in peripheral tissues by the enzyme 5'-deiodinase.

Thyroxine is the sole thyroid hormone in clinical use today. This is largely due to its availability and relatively long half-life of 6 to 7 days which results from its avid binding to thyroxine-binding globulin in human serum and its consequent protection from metabolic breakdown and excretion. Pure triiodothyronine is not in clinical use due to its relative unavailability and less than avid binding to thyroxine-binding globulin which results in a half-life of two days or less.

Thyroxine has until now been shown to play a generally negative role in heart function.

It is possible for patients to suffer either from an excess or a deficit of thyroxine. Thyroxine increases the heart rate and the force of the beats, thus increasing cardiac output, and patients suffering from hyperthyroidism, caused by an excess of thyroxine, exhibit a number of cardiac dysfunctions such as heart palpitations, dyspnea, tachycardia, systolic hypertension and a variety of heart murmurs. The effects of hyperthyroidism on the heart may also include premature beats, auricular fibrillation, increased stroke volume and increased cardiac output, and although the peripheral vascular resistance decreases, the myocardial workload becomes greater. Hyperthyroidism may ultimately lead to angina, arrhythmias and heart failure.

Thyroxine is routinely used in treatment of patients lacking adequate thyroid function. Such patients are those with hypothyroidism (myxedema), goitre or cretinism.

Due to its effect on the heart, in current practice administration of thyroxine has generally been contraindicated for patients with heart conditions such as tachyarrhythmias, acute myocardial infarction, cardiac instability and severe heart disease. Thyroxine can have serious cardiac effects even when given to patients without an underlying heart condition. Thus, Bacci et al, JAMA, 1981, 245, 920 report that a sudden, large load of thyroxine given to a hypothyroid patient with severe myxedema had a direct and rapid undesirable effect on the myocardium, causing cardiac arrest. Furthermore, thyroxine therapy for hypothyroidism has been reported by Bergeron et al, Arch. Intern. Med., 1988, 148, 1450, to have caused severe segmental left ventricular ischemic changes, subendocardial infarction and cardiogenic shock in a patient with a normal coronary anatomy.

Accordingly both in hyperthyroid patients who exhibit an excess of thyroid hormone in the body and in hypothyroid patients treated with thyroxine to correct a thyroid hormone deficiency in the body, thyroxine has been found to play a negative role in heart function. Despite this, it has now surprisingly been found that the administration of a thyroid hormone can effect cardiac resuscitation in patients undergoing cardiac arrest. The effect of the thyroid hormone is rapid and can occur even where standard treatments have failed. Thyroid hormones have been Found to affect both the chronotropic and ionotropic heart functions. This valuable function of thyroid hormones also extends into other areas than cardiac arrest, in particular the treatment of electromechanical dissociation.

Accordingly the present invention comprises the use of a thyroid hormone for the manufacture of a medicament for use in the treatment of a patient with cardiac arrest to thereby restore effective cardiac function or in cases of electromechanical dissociation.

The various investigations reported in the literature concerning hyperthyroidism, hypothyroidism and the effects of the use of thyroxine do include papers by Kranz et al, Exp. Path. Bd., 1976, 12, 129, and by Gay et al, Am. J. Physiol., 1987, 253, H341, both of which describe a beneficial effect of thyroxine on the heart. Thus, Kranz et al report experiments with hyperthyroid and hypothyroid rats in which a myocardial infarction is artificially induced. It was found that wound healing in the heart for those rats which survived the myocardial infarction was better in the hyperthyroid rats than in the hypothyroid rats. Gay et al again artificially induced a myocardial infarction in rats, but in this case normal rats were used and those rats which survived the myocardial infarction were treated with thyroxine. Studies carried out 3 weeks after the infarction showed that rats treated with low doses of thyroxine, but not with higher doses thereof, showed an improvement in LV dysfunction as compared with control rats.

Novitzky et al., European Journal of Cardiothoracic Surgery, 1989, 3, 140-145, do describe the use of triiodothyronine in patients undergoing open heart surgery following the induction of cardioplegic arrest and cardiopulmonary bypass. However, although triiodothyronine was found to be beneficial in this situation, neither this nor the other papers described above contain any indication that thyroid hormones might have value for the treatment of patients requiring cardiac resuscitation in the different situation of cardiac arrest or of electromechanical dissociation resulting post defibrillation or on myocardial infarction. It has now been found, however, that thyroxine and other thyroid hormones are effective in restoring cardiac rhythm and function in such situations even where standard treatments fail.

The term thyroid hormone used herein includes particularly compounds suitable for use in treating thyroid hormone deficiency in the body, i.e. any thyroid agonist. In general, the thyroid hormones of use in the present invention are thyroxine and triiodothyronine and derivatives and analogues thereof, either singly or in combinations of two or more thereof. Thyroxine and triiodothyronine, and their derivatives and analogues will generally be used in the L-form, which is that exhibiting a greater level of thyroid hormone activity, although, less preferably, the L-form may be used together with the D-form, for example as the DL-racemate. Typically, preferred thyroid hormones of use in the invention will have an activity which is equal to or greater than that of DL-thyroxine, particularly of L-thyroxine. Triiodothyronine and its derivatives and analogues have the advantage, among others, of a higher level of activity as compared with thyroxine and its derivatives and analogues. L-Triiodothyronine is thus the preferred thyroid hormone for use in the present invention.

The thyroid hormones thyroxine and triiodothyronine are obtainable from natural sources such as bovine thyroid glands or may be synthesized in vitro, for example using chemical methods such as those described by Anthony et al in U.S. Patent 2,803,654. Various derivatives and analogues of these compounds also exert thyroid hormone activity as defined hereinbefore and may be used in the present invention. A group of such compounds and their methods of synthesis is described by Meltzer et al in U.S. Patent 3,109,024.

If desired, the thyroxine hormone may take the form of a physiologically acceptable salt. Thyroxine and triiodothyronine contain both an amino group and a carboxy group. It is, therefore, possible to form salts with both physiologically acceptable bases and acids. Examples of suitable bases are the alkali metal hydroxides, for example sodium hydroxide, quaternary ammonium hydroxides and amines such as tris (tris representing 2-amino-2-hydroxymethyl propane 1,3-diol). Suitable acids may be inorganic or organic. Examples of such inorganic acids are phosphoric acid, nitric acid, sulphuric acid and particularly the hydrohalic acids hydrochloric acid, hydrobromic acid and hydroiodic acid. Examples of such organic acids are citric acid, oxalic acid, fumaric acid, maleic acid, lactic acid, succinic acid, malic acid, tartaric acid and methane sulphonic acid. Salts with bases are of particular interest and L-thyroxine is commonly marketed in the form of its sodium salt.

The present invention is of particular application when some degree of electrical and/or mechanical dysfunction is involved.

The use of the invention is indicated in the acute conditions of cardiac arrest and of electromechanical dissociation (EMD) resulting post defibrillation and on myocardial infarction, EMD occurring when the electrical and physical actions of the heart become dissociated so that the electrical stimulation no longer produces a concomitant physical movement.

A specific example of the application of the present invention is in the treatment of an acute condition as described hereinbefore which is induced by a cardiomyopathy. Cardiomyopathies are the result of ischemic, metabolic or idiopathic disorders or are the result of microbial infections, such as those caused by viral, bacterial, fungal or parasitic infection.

For cardiac resuscitation the thyroid hormone is administered directly into the heart cavity, parenterally or directly into the pulmonary system. In general, as an indication for injection, it may be stated that wherever epinephrine was previously utilized, thyroid hormones may now be used with advantage either to replace the epinephrine or in conjunction with it. Such cardiac resuscitation treatment is employed in the treatment of the acute conditions of cardiac arrest and electromechanical dissociation as described hereinbefore.

Examples of these modes of administration are as follows. Direct administration into the heart may particularly involve direct intracardiac injection. Parenteral administration may involve a central venous line infusion via a pump or direct intravenous injection. Pulmonary administration may involve direct endotracheal injection (or infusion), such as through an endotracheal tube, or through an airway system, such as through a vaporizer, atomizer or an endotracheal tube.

The preferred dosage of thyroid hormone depends particularly on the specific activity of the thyroid hormone used. The dosage ranges may, however, generally be at a somewhat higher level than those usually employed when the hormones are used in other contexts such as the treatment of hypothyroidism. Thus, for example, when used in treating hypothyroid patients L-thyroxine is usually given orally in pill form at a unit dosage level of 25 to 200 »g. However, in cases where oral administration of thyroxine is not possible or in an emergency such as where hypothyroidism has led to coma, L-thyroxine is administered parenterally, either intravenously or by intramuscular injection. Parenteral administration is usually at a similar dosage to that taken orally except in the case of emergency where up to 200 to 500 »g may be administered intravenously.

Although the dosages preferred for the medicament of the invention will vary even in the case of a particular thyroid hormone, depending on the condition being treated, it may be stated by way of guidance that in the case of L-thyroxine the dose for a human patient will often be in a range of 100 »g to 10 g when given parenterally in at least one rapid bolus injection with repeated injections of comparable amounts as necessary to attain and sustain hemodynamic stability. In the case of L-triiodothyronine the range for similar usage will usually be 1 »g to 1 mg. Veterinary usage, for example in mammals, will be on a similar ratio of thyroid hormone/body weight. Appropriate doses of other compounds may be calculated according to their relative activity to L-thyroxine and L-triiodothyronine in standard uses of these compounds. Variations within these ranges of dosages for the thyroid hormones will depend on the weight of the patient, the severity of the situation, the underlying pathology and the time from onset of the cardiac arrest with greater amounts being given as the time which has elapsed from cardiac arrest increases. However, a more commonly used minimum dosage is 500 »g or 1 mg for L-tyroxine and 50 »g or 100 »g for L-triiodothyronine whilst a more common maximum for L-tyroxine will be somewhat less than 10 g, for example 1 g or 100 mg. It is preferred that the patient undergoing cardiac resuscitation receives the thyroid hormone in conjunction with defibrillation although thyroid hormones may be administered in the absence of other therapy.

Overdoses of thyroid hormones can if necessary be immediately aborted with intravenous doses of β-blockers, for example propanolol and metoprolol. Furthermore, when L-thyroxine is used in large doses subsequent treatment with β-blockers may be appropriate to prevent the effects of the induced hyperthyroid condition on the heart. On the other hand, if L-triiodothyronine is used, β-blockers may not be necessary or may be required only at lower levels as it is rapidly metabolized and/or excreted from the body. Thus, even though it has not heretofore been used clinically, L-triiodothyronine and its derivatives and analogues are preferred over L-thyroxine and its derivatives and analogues as the former are more active on a molar basis and give rise to fewer hyperthyroid symptoms. Thus L-triiodothyronine is approximately four times as potent as L-thyroxine whilst the 3'-isopropyl-3,5-diiodothyronine analogue is approximately seven times as potent.

For the most part, the medicaments prepared according to the present invention may take conventional forms, and may be formulated in unit dosage form where desired, i.e. in the form of discrete portions each comprising a unit dose, or a multiple or sub-multiple of a unit dose, for example 100 »g, 500 »g, 1 mg, 10 mg, 100 mg, 1 g or more for L-thyroxine and 50 »g, 100 »g, 250 »g, 500 »g or more for triiodothyronine.

In general, the medicament may incorporate a liquid diluent or carrier, for example an aqueous or oily suspension, emulsion or particularly solution, which may often be employed in injectable or infusable form for parenteral administration or pulmonary administration and therefore may conveniently be sterile and pyrogen free. For parenteral use thyroxine is preferably supplied in lyophilized form and is reconstituted, for example with saline, immediately prior to use.

The present invention may utilise a kit suitable for the administration of a thyroid hormone to a patient undergoing cardiac arrest. Such a kit comprises a thyroid hormone in a physiologically acceptable liquid diluent or carrier or in a solid form suitable for formulation in such a liquid diluent or carrier prior to use (which liquid diluent or carrier may then optionally form a separate part of the kit) together with a device for the injection of the thyroid hormone. The solid form may conveniently consist of powdered or lyophilized material. The liquid diluent or carrier may, for example, be saline or another liquid which provides a solution of the hormone and is physiologically acceptable. An example of a suitable injection device is one based on the Abboject "Unit of Use Syringe" supplied by Abbott Laboratory which delivers a single dose of adrenalin to the heart via an intracardiac needle.

The present invention further include a kit for treatment of the acute condition of cardiac arrest in a patient which comprises a thyroid hormone, a physiologically acceptable liquid diluent or carrier and an intracardiac syringe for administering the thyroid hormone to the heart of the patient.

As indicated previously, the medicament can contain more than one thyroid hormone and, in addition, may optionally contain other substances which are therapeutically effective in enhancing the function of the heart, for example a suitable formulation of one or both of magnesium and calcium. It is also possible for the thyroid hormone to be used in conjunction with epinephrine and if desired the two compounds may be formulated together.

The invention is illustrated by the following Examples which describe the use of L-thyroxine to treat dogs in which cardiac arrest has been artifically induced. It will be seen from the examples that L-thyroxine restored normal cardiac function even where standard methods had failed and did not cause any symptoms of hyperthyroidism in the treated dogs. However, since dogs lack thyroxine-binding globulin, L-thyroxine is rapidly metabolized or excreted after administration. In humans, L-thyroxine persists up to a week after treatment and for this reason its effects are often counteracted by β-blockers administered subsequently to the thyroxine treatment. On the other hand, L-triiodothyronine does not bind avidly to thyroxine-binding globulin and the effect in humans of this preferred thyroid hormone thus directly correlates with the observed effect of L-thyroxine in dogs.

### Example 1

Using a Ventritex bedside, external pulse generator and defibrillator, rapid pacing at a rate of 30-50 milliseconds for approximately 4-6 seconds was used to induce ventricular fibrillation in a mongrel dog weighing 30-50 pounds.

Defibrillation threshold in the dog was established by repeated attempts at fibrillation and defibrillation via the Ventritex pulse generator according to the manufacturer's instructions. During one episode while attempting to determine threshold defibrillation, standard shock (250 volts (V)) and "rescue shock" (950 V) were ineffective. Large energy pulses were then applied via an external defibrillator (Hewlett Packard), resulting in cardiac standstill as determined by electrocardiographic monitoring. The dog was then paced with 10 V of 1 millisecond duration at a rate of 100 beats per minute (bpm).

Every attempt at turning off the pacing unit resulted in returning the rhythm to standstill as evidenced by ECG and lack of palpable pulse. After approximately three minutes of cardiac standstill without any effective rhythm, the dog was given an intravenous bolus of 250 »g of L-thyroxine (obtained from Stris Laboratories Inc., Arizona, and prepared according to the manufacturer's instructions). Approximately 1-1.5 minutes after receiving the L-thyroxine the dog reverted to normal cardiac rhythm with a good palpable pulse.

### Example 2

After establishing defibrillation threshold as described in Example 1, external defibrillation was administered eight times as in Example 1, without establishing normal cardiac rhythm. The dog was then given an intravenous bolus injection of 250 »g of L-thyroxine. Approximately 1 minute after receiving the L-thyroxine the dog developed a spontaneous rhythm alternating between sinus and supraventricular. The dog subsequently developed A-V dissociation, and was given a second intravenous bolus injection of 250 »g of L-thyroxine upon which the dog reverted to normal cardiac rhythm with an effective pulse. The dog maintained a sinus rhythm and good pulse until it was sacrificed.

### Example 3

A dog was put into fibrillation and the defibrillation threshold was obtained as described in Example 1. Throughout the experiment the dog was on a ventilator to maintain oxygenation. Defibrillation was attempted at 250 V without effect. A rescue shock of 950 V was then applied as in Example 1 without effect. The 950 V shock was repeated 23 times without effect. At this point the dog's chest was opened and a shock was applied directly to the heart with internal epicardial paddles at 400 joules using a Hewlett Packard defibrillator as per the manufacturer's instructions.

After the direct cardiac stimulation the dog briefly came out of fibrillation but immediately returned to ventricular fibrillation with no apparent mechanical activity. The heart was again stimulated with internal epicardial paddles at 400 joules and the dog reverted to a tachyarrhythmia with electromechanical dissociation which then degenerated to ventricular fibrillation. The dog was then shocked with 950 V externally and defibrillation persisted. At this point, about 8.5 minutes after initial onset of fibrillation, L-thyroxine was administered in a bolus of 250 »g given by intracardiac injection. After approximately 100 seconds and four defibrillation attempts the dog developed atrioventricular dissociation and subsequently reverted to a super ventricular tachyardia with electromechanical dissociation. A second intravenous bolus of 250 »g of L-thyroxine was then administered and in less than 20 seconds the dog developed an effective pulse and rapidly reverted back to a sinus rhythm. The following morning the dog was alert and ate well.

## Claims

1. The use of a thyroid hormone for the manufacture of a medicament for use in treatment of the acute condition of cardiac arrest in a patient to thereby restore effective cardiac function.

2. The use of a thyroid hormone for the manufacture of a medicament for use in treatment of the acute condition of electromechanical dissociation in a patient following myocardial infarction to thereby establish a cardiac rhythm and restore effective cardiac function.

3. The use of a thyroid hormone for the manufacture of a medicament for use in treatment of the acute condition of electromechanical dissociation in a patient in whom defibrillation has failed to establish a cardiac rhythm, to thereby establish a cardiac rhythm and restore effective cardiac function.

4. The use according to Claim 1, 2 or 3, in which the administration of the thyroid hormone establishes normal sinus rhythm.

5. The use according to any of Claims 1 to 4, in which the acute condition is caused by a cardiomyopathy.

6. The use according to any of Claims 1 to 5, in which the patient is treated with the medicament by direct injection into a heart cavity.

7. The use according to any of Claims 1 to 5, in which the patient is treated with the medicament by parenteral injection.

8. The use according to Claim 7, in which the parenteral injection is intravenous.

9. The use according to Claim 7, in which the parenteral injection is into a central venous line of the patient.

10. The use according to any of Claims 1 to 5, in which the patient is treated with the medicament by administration directly to the pulmonary system.

11. The use according to Claim 8, in which the administration is by direct endotracheal injection.

12. The use according to Claim 8, in which the administration is by infusion through a respiratory airway.

13. The use according to any of Claims 1 to 6 and 9, in which the medicament is administered in at least one rapid bolus injection.

14. The use according to any of the preceding claims, in which the thyroid hormone is thyroxine or triiodothyronine or an analogue or derivative thereof.

15. The use according to any of Claims 1 to 13, in which the thyroid hormone is thyroxine.

16. The use according to Claim 15, in which the patient is treated with a dose of L-thyroxine in the range of 500 »g to 10 g.

17. The use according to any of Claims 1 to 13, in which the thyroid hormone is triiodothyronine.

18. The use according to Claim 17, in which the patient is treated with a dose of L-triiodothyronine in the range of 50 »g to 1 mg.

19. The use according to any of the preceding claims, in which the medicament contains, as a further active component thereof, one or both of magnesium and calcium.

20. The use according to any of the preceding claims, in which the medicament is presented as a kit comprising the thyroid hormone, a physiologically acceptable diluent or carrier and means for administering the thyroid hormone in the diluent or carrier to the patient by injection.

21. A kit for treatment of the acute condition of cardiac arrest in a patient which comprises a thyroid hormone, a physiologically acceptable liquid diluent or carrier and an intracardiac syringe for administering the thyroid hormone to the heart of the patient.

22. A kit for the treatment of the acute condition of cardiac arrest in a patient which comprises a thyroid hormone in solid form, a separate liquid diluent or carrier and an intracardiac syringe for the injection of a mixture of the thyroid hormone and the liquid diluent or carrier into the heart of the patient.

23. A kit according to Claim 21 or 22, in which the thyroid hormone is thyroxine or triiodothyronine or an analogue or derivative thereof.

24. A kit according to Claim 23, in which the hormone is thyroxine.

25. A kit according to Claim 24, which contains a dose of L-thyroxine in the range of 500 »g to 10 g.

26. A kit according to Claim 23, in which the hormone is triiodothyronine.

27. A kit according to Claim 26, which contains a dose of L-triiodothyronine in the range of 50 »g to 1 mg.

28. A kit according to any of Claims 21 to 27 which additionally contains one or both of magnesium and calcium.

## Patentansprüche

1. Verwendung eines Schilddrüsenhormons zur Herstellung eines Medikaments zur Verwendung bei der Behandlung des akuten Zustands des Herzstillstands bei einem Patienten, um dadurch die effektive Herzfunktion wiederherzustellen.

2. Verwendung eines Schilddrüsenhormons zur Herstellung eines Medikaments zur Verwendung bei der Behandlung des akuten Zustands der elektromechanischen Dissoziation bei einem Patienten nach einem Myocardinfarkt, um dadurch einen Herzrhythmus herzustellen und die effektive Herzfunktion wiederherzustellen.

3. Verwendung eines Schilddrüsenhormons zur Herstellung eines Medikaments zur Verwendung bei der Behandlung des akuten Zustands der elektromechanischen Dissoziation bei einem Patienten, bei dem die Defibrillation nicht den Herzrhythmus herstellen konnte, um dadurch einen Herzrhythmus herzustellen und die effektive Herzfunktion wiederherzustellen.

4. Verwendung nach Anspruch 1, 2 oder 3, wobei die Verabreichung des Schilddrüsenhormons den normalen Sinusrhythmus herstellt.

5. Verwendung nach einem der Ansprüche 1 bis 4, wobei der akute Zustand durch eine Cardiomyopathie hervorgerufen wird.

6. Verwendung nach einem der Ansprüche 1 bis 5, wobei der Patient mit dem Medikament durch direkte Injektion in den Herzinnenraum behandelt wird.

7. Verwendung nach einem der Ansprüche 1 bis 5, wobei der Patient mit dem Medikament durch parenterale Injektion behandelt wird.

8. Verwendung nach Anspruch 7, wobei die parenterale Injektion intravenös erfolgt.

9. Verwendung nach Anspruch 7, wobei die parenterale Injektion in die Zentralvene des Patienten erfolgt.

10. Verwendung nach einem der Ansprüche 1 bis 5, wobei der Patient mit dem Medikament durch direkte Verabreichung in das Lungensystem behandelt wird.

11. Verwendung nach Anspruch 8, wobei die Verabreichung durch direkte endotracheale Injektion erfolgt.

12. Verwendung nach Anspruch 8, wobei die Verabreichung durch Infusion durch einen Atemweg erfolgt.

13. Verwendung nach einem der Ansprüche 1 bis 6 und 9, wobei das Medikament in mindestens einer raschen Bolusinjektion verabreicht wird.

14. Verwendung nach einem der vorausgehenden Ansprüche, wobei das Schilddrüsenhormon Thyroxin oder Triiodthyronin oder ein Analogon oder ein Derivat davon ist.

15. Verwendung nach einem der Ansprüche 1 bis 13, wobei das Schilddrüsenhormon Thyroxin ist.

16. Verwendung nach Anspruch 15, wobei der Patient mit einer L-Thyroxindosis im Bereich von 500 »g bis 10 g behandelt wird.

17. Verwendung nach einem der Ansprüche 1 bis 13, wobei das Schilddrüsenhormon Triiodthyronin ist.

18. Verwendung nach Anspruch 17, wobei der Patient mit einer L-Triiodthyronindosis im Bereich von 50 »g bis 1 mg behandelt wird.

19. Verwendung nach einem der vorausgehenden Ansprüche, wobei das Medikament als weiteren Wirkstoff Magnesium und/oder Calcium enthält.

20. Verwendung nach einem der vorausgehenden Ansprüche, wobei das Medikament als Kit, umfassend das Schilddrüsenhormon, ein physiologisch verträgliches Verdünnungsmittel oder einen physiologisch verträglichen Träger und Mittel zur Verabreichung des Schilddrüsenhormons in dem Verdünnungsmittel oder Träger an den Patienten durch Injektion, bereitgestellt wird.

21. Kit zur Behandlung des akuten Zustands des Herzstillstands bei einem Patienten, umfassend ein Schilddrüsenhormon, ein physiologisch verträgliches flüssiges Verdünnungsmittel oder Träger und eine intracardiale Spritze zur Verabreichung des Schilddrüsenhormons an das Herz des Patienten.

22. Kit zur Behandlung des akuten Zustands des Herzstillstands bei einem Patienten, umfassend ein Schilddrüsenhormon in fester Form, ein getrenntes flüssiges Verdünnungsmittel oder einen Träger und eine intracardiale Spritze zur Injektion eines Gemisches des Schilddrüsenhormons und des flüssigen Verdünnungsmittels oder Trägers in das Herz des Patienten.

23. Kit nach Anspruch 21 oder 22, worin das Schllddrüsenhormon Thyroxin oder Triiodthyronin oder ein Analogon oder Derivat davon ist.

24. Kit nach Anspruch 23, worin das Hormon Thyroxin ist.

25. Kit nach Anspruch 24, welches eine L-Thyroxindosis im Bereich von 500 »g bis 20 g enthält.

26. Kit nach Anspruch 23, worin das Hormon Triiodthyronin ist.

27. Kit nach Anspruch 26, welches eine L-Triiodthyronindosis im Bereich von 50 »g bis 1 mg enthält.

28. Kit nach einem der Ansprüche 21 bis 27, welches zusätzlich Magnesium und/oder Calcium enthält.

## Revendications

1. Utilisation d'une hormone thyroïdienne dans la préparation d'un médicament utilisable dans le traitement de la phase aiguë d'un arrêt cardiaque chez un patient, afin de restaurer la fonction cardiaque normale.

2. Utilisation d'une hormone thyroïdienne dans la préparation d'un médicament utilisable dans le traitement de la phase aiguë d'une dissociation électromécanique chez un patient, à la suite d'un infarctus du myocarde, afin d'établir un rythme cardiaque et de restaurer la fonction cardiaque normale.

3. Utilisation d'une hormone thyroïdienne dans la préparation d'un médicament utilisable dans le traitement de la phase aiguë d'une dissociation électromécanique chez un patient chez qui la défibrillation n'a pas permis d'établir un rythme cardiaque, afin d'établir un rythme cardiaque et de restaurer la fonction cardiaque normale.

4. Utilisation selon la revendication 1, 2 ou 3, dans laquelle l'administration d'une hormone thyroïdienne établit un rythme sinusoïdal normal.

5. Utilisation selon l'une quelconque des revendications 1 à 4, dans laquelle la phase aiguë est due d'une cardiomyopathie.

6. Utilisation selon l'une quelconque des revendications 1 à 5, dans laquelle le patient est traité avec le médicament par injection directe dans la cavité cardiaque.

7. Utilisation selon l'une quelconque des revendications 1 à 5, dans laquelle le patient est traité avec le médicament par injection parentérale.

8. Utilisation selon la revendication 7, dans laquelle l'injection parentérale se fait par voie intraveineuse.

9. Utilisation selon la revendication 7, dans laquelle l'injection parentérale se fait dans une veine centrale du patient.

10. Utilisation selon l'une quelconque des revendications 1 à 5, dans laquelle le patient est traité avec le médicament par administration directe dans le système pulmonaire.

11. Utilisation selon la revendication 8, dans laquelle l'administration se fait par injection endotrachéale directe.

12. Utilisation selon la revendication 8, dans laquelle l'administration se fait par inhalation via les voies respiratoires.

13. Utilisation selon l'une quelconque des revendications 1 à 6 et 9, dans laquelle le médicament est administré par injection d'au moins un embol rapide.

14. Utilisation selon l'une quelconque des revendications précédentes, dans laquelle l'hormone thyroïdienne est la thyroxine ou la triiodothyronine ou un analogue ou un dérivé de celles-ci.

15. Utilisation selon l'une quelconque des revendications 1 à 13, dans laquelle l'hormone thyroïdienne est la thyroxine.

16. Utilisation selon la revendication 15, dans laquelle le patient est traité avec une dose de L-thyroxine allant de 500 »g à 10 g.

17. Utilisation selon l'une quelconque des revendications 1 à 13, dans laquelle l'hormone thyroïdienne est la triiodothyronine.

18. Utilisation selon la revendication 17, dans laquelle le patient est traité avec une dose de L-triidothyronine allant de 50 »g à 1 mg.

19. Utilisation selon l'une quelconque des revendications précédentes, dans laquelle le médicament contient, en tant qu'autre constituant actif, du magnésium ou du calcium ou les deux.

20. Utilisation selon l'une quelconque des revendications précédentes, dans laquelle le médicament est présenté sons forme de kit comprenant l'hormone thyroïdienne, un véhicule ou un diluant physiologiquement acceptable et des moyens pour administrer par injection au patient l'hormone thyroïdienne dans le véhicule ou le diluant.

21. Kit pour le traitement de la phase aiguë d'un arrêt cardiaque chez un patient, comprenant une hormone thyroïdienne, un véhicule ou un diluant liquide physiologiquement acceptable et une seringue intracardiaque pour administrer l'hormone thyroïdienne dans le coeur du patient.

22. Kit pour le traitement de la phase aiguë d'un arrêt cardiaque chez un patient, comprenant une hormone thyroïdienne sous forme solide, un véhicule ou un diluant liquide séparé et une seringue intracardiaque pour l'injection d'un mélange de l'hormone thyroïdienne et du véhicule ou diluant liquide dans le coeur du patient.

23. Kit selon la revendication 21 ou 22, dans lequel l'hormone thyroïdienne est la thyroxine ou la triiodothyronine ou un analogue ou un dérivé de celles-ci.

24. Kit selon la revendication 23, dans lequel l'hormone est la thyroxine.

25. Kit selon la revendication 24, qui contient une dose de L-thyroxine allant de 500 »g à 10 g.

26. Kit selon la revendication 23, dans lequel l'hormone est la triiodothyronine.

27. Kit selon la revendication 26, qui contient une dose de L-triiodothyronine allant de 50 »g à 1 mg.

28. Kit selon l'une quelconque des revendications 21 à 27 qui contient en outre du magnésium ou du calcium ou les deux.
